(19) Europäisches Patentamt
European Patent Office
Office européen des brevets

(11) Numéro de publication : **0 667 344 A1**

# (12) DEMANDE DE BREVET EUROPEEN

(21) Numéro de dépôt : **95400293.7**

(22) Date de dépôt : **13.02.95**

(51) Int. Cl.[6] : **C07D 403/12**, C07D 243/24, C07D 401/12, A61K 31/55

(30) Priorité : **14.02.94 FR 9401642**
**12.07.94 FR 9408665**

(43) Date de publication de la demande :
**16.08.95 Bulletin 95/33**

(84) Etats contractants désignés :
**AT BE CH DE DK ES FR GB GR IE IT LI LU MC NL PT SE**

(71) Demandeur : **SANOFI**
**32-34, rue Marbeuf**
**F-75008 Paris (FR)**

(72) Inventeur : **Badorc, Alain**
**1, rue Lacanal**
**F-31120 Roquettes (FR)**
Inventeur : **Despeyroux, Pierre**
**190, avenue du Comminges - Labarthe/Leze**
**F-31120 Portet/Garonne (FR)**
Inventeur : **Gully, Danièle**
**82, route de Roquettes,**
**Saubens**
**F-31600 Muret (FR)**
Inventeur : **De Cointet, Paul**
**6, rue Darquier**
**F-31000 Toulouse (FR)**
Inventeur : **Frehel, Daniel**
**Rés. de l'Autan,**
**Appt. 206-100, allée de Barcelone**
**F-31100 Toulouse (FR)**
Inventeur : **Maffrand, Jean-Pierre**
**5, rue du Corps Franc Pomiès**
**F-31120 Mortet/Garonne (FR)**

(74) Mandataire : **Le Guen, Gérard et al**
**CABINET LAVOIX**
**2, place d'Estienne d'Orves**
**F-75441 Paris Cédex 09 (FR)**

(54) **3-acylamino-5-(phényl polysubstitué)-1,4-benzodiazépin-2-ones utiles comme abonistes du récepteur CCK.**

(57) L'invention concerne des composés de formule (I)

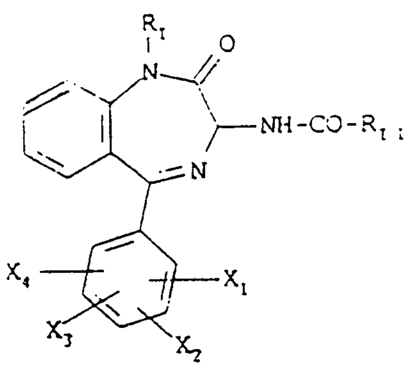

(I)

dans laquelle

— $R_I$, représente l'hydrogène ou un substituant,

— $R_{II}$ représente soit (i) un hétérocycle aromatique azoté, $R_{IIa}$, choisi parmi quinoléine, isoquinoléine, benzimidazole et indole, ce dernier étant éventuellement substitué sur l'azote par un groupe W où W représente le groupe CO-($C_1$-$C_4$)alkyle ; ou le groupe $(CH_2)_nCOR_O$ dans lequel n et $R_O$ sont tels que définis pour $R_I$ ; soit (ii) un groupe

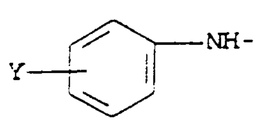

($R_{IIb}$)

EP 0 667 344 A1

Jouve, 18, rue Saint-Denis, 75001 PARIS

dans lequel Y représente un atome d'halogène, un groupe ($C_1$-$C_3$) alkyle, ou ($C_1$-$C_3$) alcoxy ;

— $X_1$, $X_2$ et $X_3$ sont identiques ou différents et représentent un ($C_1$-$C_3$) alkyle, un ($C_1$-$C_3$) alcoxy, un atome d'halogène ou un groupe trifluorométhyle ;

— $X_4$ représente l'hydrogène ou est identique à $X_1$, $X_2$ ou $X_3$, leurs stéréosiomères et leurs sels d'addition.

Ces composés sont utiles comme agonistes du recepteur CCK.

La présente invention concerne des dérivés de 3-acylamino-5-phényl-1,4-benzodiazépin-2-one ainsi que leurs sels, leur procédé de préparation et les compositions pharmaceutiques les contenant.

Les composés de la présente invention se distinguent d'autres dérivés de 1,4-benzodiazépines, par leur structure originale et par leurs propriétés pharmacologiques nouvelles. Il s'agit de dérivés de 1,4-benzodiazépines dont le phényle en position 5 de la benzodiazépine est au moins trisubstitué.

Cette structure particulière confère aux produits selon l'invention des propriétés pharmacologiques inattendues et très intéressantes. En effet, les composés de l'invention ont une affinité pour le récepteurs de la cholécystokinine plus particulièrement, la présente invention a pour objet de nouveaux agonistes des récepteurs de la cholécystokinine (CCK) sur le test de l'amylase pancréatique.

La CCK est un peptide largement distribué dans le cerveau, notamment dans le cortex, le striatum, l'hippocampe, le tegmentum ventral, le septum et l'hypothalamus.

La CCK est également secrétée au niveau périphérique par l'intestin grêle, son action se manifeste notamment par la stimulation de la contraction vésiculaire, une augmentation de la sécrétion biliaire, le contrôle de la sécrétion enzymatique pancréatique, une action sur les contractions gastriques, une action sur la motilité intestinale. Elle pourrait agir dans certains cas sur la pression artérielle et influencer les systèmes immunitaires.

La CCK coexiste dans certains neurones centraux avec la dopamine. Elle intervient également dans des mécanismes impliquant l'acétylcholine, le gaba, la sérotonine, les opioïdes, la somatostatine, la substance P et des canaux ioniques.

Son administration provoque des modifications physiologiques: ptose palpébrale, hypothermie, hyperglycémie, catalepsie, et comportementales : hypolocomotricité, diminution de l'exploration, analgésie, action dans les apprentissages, modification du comportement sexuel et satiété.

Un agoniste du récepteur de la CCK, notamment du récepteur CCKA, peut donc être utilisé comme médicament dans le traitement de certains troubles du comportement alimentaire, de l'obésité, du diabète, dans les troubles du comportement émotionnel, sexuel et mnésique, dans la schizophrénie, dans les psychoses, dans la maladie de Parkinson et dans divers troubles de la sphère gastrointestinale (Drugs of the Future, 1992, *17*, n° 3, 197-206).

Jusqu'à ce jour, toutes les 1,4-benzodiazépines décrites ayant une bonne affinité pour les récepteurs CCKA sont des antagonistes.

Des composés ligands des récepteurs de la CCK et de la gastrine sont connus dans la littérature, la demande de brevet WO-93/20099, par exemple l'illustre.

On peut encore citer, à titre d'exemple, le Devazépide ou le L-365260 dont les formules sont respectivement

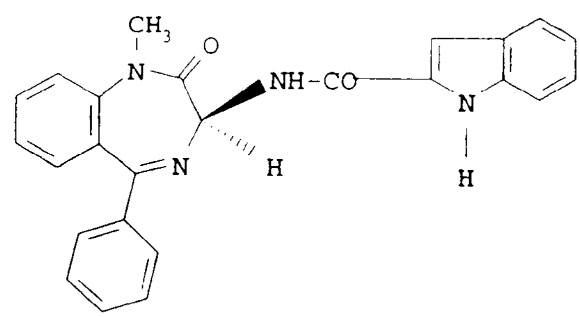

(A)

Devazépide

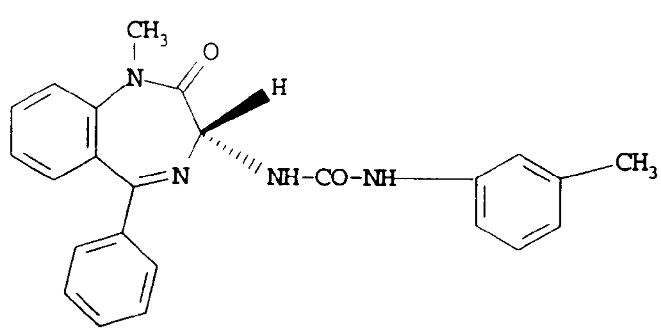

(B)

L-365260

Des agonistes du récepteur de la CCK de nature peptidique ou de structure très différente des composés selon la présente invention sont décrits dans la littérature. Par exemple, certains produits ayant de telles propriétés sont décrits dans EP-A-0383690, WO 90/06937 et EP-A-0376849.

Les composés de l'invention ainsi que leurs sels d'addition avec les acides minéraux ou organiques répondent à la formule :

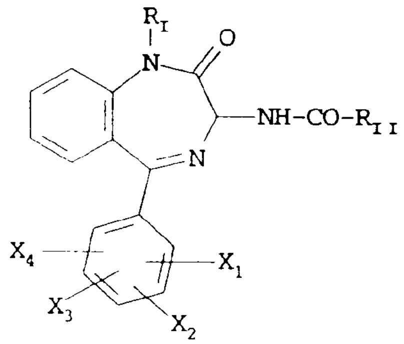

(I)

dans laquelle

- $R_I$ représente
  (i) l'hydrogène ;
  (ii) un ($C_1$-$C_4$) alkyle;
  (iii) un groupe $-CH_2-CHOH-(CH_2)_m-Z$ dans lequel
  m est 0 ou 1 et Z représente un groupe alkyle en ($C_1$-$C_4$); cycloalkyle en ($C_3$-$C_8$); aryle éventuellement substitué par ($C_1$-$C_4$) alkyle, ($C_1$-$C_4$) alkoxy ou halogène; ou Z représente un hétérocycle saturé ou non, éventuellement substitué par ($C_1$-$C_4$) alkyle, ($C_1$-$C_4$) alkoxy ou halogène; ou
  (iv) $R_I$ représente un groupe $-(CH_2)_nCOR_o$ dans lequel
  n représente 1 à 3,
  $R_o$ représente $OR_2$ ou $NR_2R_3$, $R_2$ et $R_3$ identiques ou non représentant H ou ($C_1$-$C_4$) alkyle ou bien $R_2$ et $R_3$ constituant ensemble avec l'atome d'azote auquel ils sont liés un hétérocycle saturé ou non;
- $R_{II}$ représente
  soit (i) un hétérocycle aromatique azoté, $R_{IIa}$, choisi parmi quinoléine, isoquinoléine, benzimidazole et indole, ce dernier étant éventuellement substitué sur l'azote par un groupe W où W représente le groupe CO-($C_1$-$C_4$)alkyle; ou le groupe $(CH_2)_nCOR_o$ dans lequel n et $R_o$ sont tels que définis pour $R_I$;
  soit (ii) un groupe

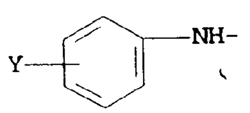

($R_{IIb}$)

  dans lequel Y représente un atome d'halogène, un groupe ($C_1$-$C_3$) alkyle, ou ($C_1$-$C_3$) alcoxy;
- $X_1$, $X_2$ et $X_3$ sont identiques ou différents et représentent un ($C_1$-$C_3$) alkyle, un ($C_1$-$C_3$) alcoxy, un atome d'halogène ou un groupe trifluorométhyle;
- $X_4$ représente l'hydrogène ou est identique à $X_1$, $X_2$ ou $X_3$,
  et leurs stéréoisomères.

Par le terme radical alkyle, on entend des radicaux alkyle de 1 à 4 atomes de carbone, linéaires ou ramifiés.

Par le terme hétérocycle saturé ou non, on entend un hétérocycle aromatique ou non aromatique choisi parmi furanne, thiophène, pyrrole, imidazole, pyrrolidine, pipéridine, pipérazine, pyridine ou morpholine.

Un groupe particulier de composés selon l'invention est constitué des composés de formule I dans lesquels $X_1$ est en position 2, $X_2$ est en position 4, $X_3$ est en position 6 et $X_4$ est indifféremment en position 3 ou 5 du radical phényle.

Parmi ces composés, on préfère ceux dans lesquels au moins trois des substituants $X_1$, $X_2$, $X_3$ ou $X_4$ représentent ($C_1$-$C_2$) alkyle ou ($C_1$-$C_2$) alcoxy et en particulier ceux choisis parmi les groupes méthyle ou méthoxy en position 2, 4, 6 du noyau aromatique; par ailleurs, on préfère que $R_{IIa}$ représente indolyle, substitué ou non.

Un autre groupe de composés préférés est constitué des composés dans lesquels deux des substituants $X_1$, $X_2$, $X_3$ et $X_4$ représentent respectivement un groupe 2-($C_1$-$C_3$)alcoxy et 6-($C_1$-$C_3$) alcoxy.

Lorsque $X_1$, $X_2$ ou $X_3$ sont un atome d'halogène, il s'agit de préférence de l'atome de chlore, mais ils peuvent également représenter un atome de fluor, de brome ou d'iode.

Les composés selon l'invention possèdent tous un centre d'asymétrie et peuvent donc ainsi exister sous forme d'isomères optiques. La présente invention comprend aussi bien ces isomères soit séparément soit en tant que mélange.

Les sels d'addition avec les acides minéraux ou organiques peuvent être, par exemple, les sels formés avec les acides chlorhydrique, bromhydrique, nitrique, sulfurique, phosphorique, acétique, formique, propionique, benzoïque, maléique, fumanque, succinique, tartrique, citrique, oxalique, glyoxylique, aspartique, alcane sulfoniques tels que les acides méthane ou éthane sulfoniques, arylsulfoniques, tels que les acides benzène ou paratoluènesulfoniques et arylcarboxyliques.

Parmi les produits de l'invention, on peut citer notamment les dérivés répondant à la formule (I) ci-dessus, ainsi que leurs sels d'addition avec les acides minéraux ou organiques, caractérisés en ce que dans ladite formule (I), le substituant $R_{II}$ représente un groupe $R_{IIa}$ de formule

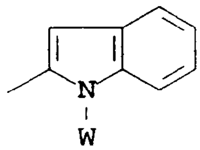

et dans laquelle W, $X_1$, $X_2$, $X_3$ et $X_4$ ont la signification précédente.

Parmi les composés préférés de l'invention, on peut citer les composés suivants :

La N-[2,3-dihydro-5-(2,6-diméthoxy-4-méthylphényl)-1-méthyl-2-oxo-1*H*-1,4-benzodiazépin-3-yl]-1H-2-indolecarboxamide ;

l'acide [N-[2,3-dihydro-5-(2,6-diméthoxy-4-méthylphényl)-1-méthyl-2-oxo-1*H*-1,4-benzodiazépin-3-yl]-2-aminocarbonyl-1H-indol-1-yl]acétique ;

la {N-[2,3-dihydro-5-(2,6-diméthoxy-4-méthylphényl)-1-méthyl-2-oxo-1*H*-1,4-benzodiazépin-3-yl]-2-aminocarbonylindol-1-yl}acétate de méthyle ;

la N-[2,3-dihydro-5-(2,6-diméthoxy-4-méthylphényl)-1-méthyl-2-oxo-1*H*-1,4-benzodiazépin-3-yl]-1*H*-indol-2-carboxamide dextrogyre ;

la N-[2,3-dihydro-5-(2,3,6-triméthoxy-4-méthylphényl)-1-méthyl-2-oxo-1*H*-1,4-benzodiazépin-3-yl]-1*H*-2-indolecarboxamide

Les composés selon l'invention peuvent être préparés selon le SCHEMA 1 suivant :

**SCHEMA 1**

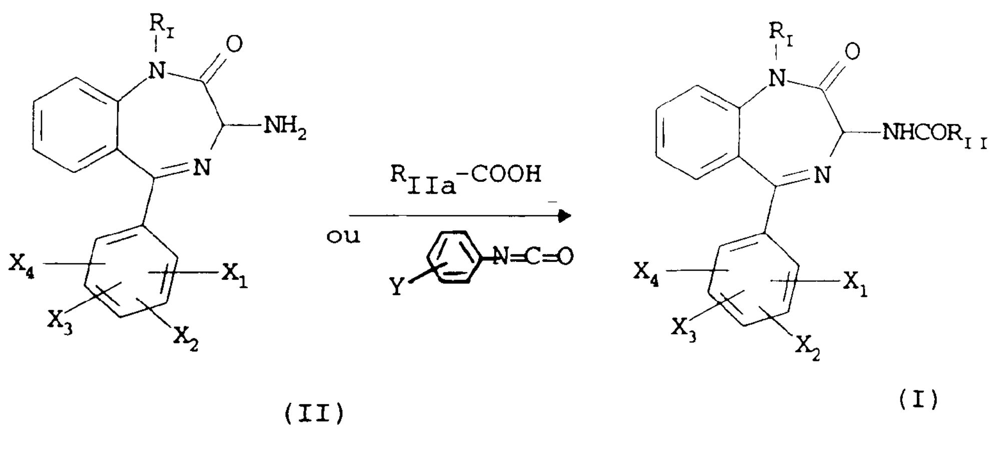


Lorsque $R_{II}$ représente un hétérocycle azoté $R_{IIa}$, les composés de formule (I) peuvent être préparés par condensation d'une 3-amino-5-phényl(substitué)-1-méthyl-1,4-benzodiazépin-2-one de formule (II) dans les conditions habituelles d'acylation d'une fonction amine avec un acide de formule $R_{IIa}COOH$ dans lequel les fonctions sensibles de $R_{IIa}$ ont été éventuellement protégées, ou avec une forme activée de l'acide telle qu'un halogénure d'acide, un anhydride d'acide ou un ester activé obtenu avec les réactifs couramment utilisés en synthèse peptidique.

Lorsque des fonctions ont été protégées, on effectue après la condensation la réaction de déprotection convenable, si nécessaire.

Certains des acides $R_{IIa}COOH$ sont connus et même commerciaux les autres sont préparés en utilisant les méthodes connues pour des molécules analogues notamment selon les méthodes décrites dans EP-A432040.

Lorsque $R_{II}$ représente un groupe $R_{IIb}$

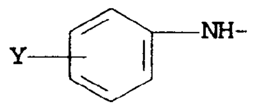

($R_{IIb}$)

dans lequel Y est tel que défini pour (I), les urées correspondantes (I) peuvent être préparées à partir de l'aminobenzodiazépine (II) avec un phénylisocyanate substitué de formule

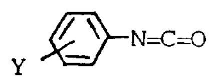

dans lequel Y est tel que défini précédemment. Ces dérivés isocyanate sont disponibles commercialement ou peuvent être préparés par des méthodes connues.

Les 3-amino-1,4-benzodiazépin-2-one de formule (II) ainsi que les intermédiaires de synthèse (V), (VI) et (VII) ci-après sont nouveaux et représentent un autre aspect de la présente invention.

Selon un autre de ses aspects, la présente invention concerne un procédé pour la préparation des composés de formule (I) caractérisé en ce que l'on fait réagir un dérivé benzénique substitué de formule

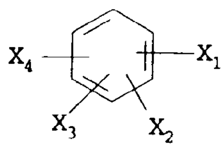

(III)

dans laquelle $X_1$, $X_2$, $X_3$ et $X_4$ sont tels que définis pour (I) avec un anhydride isatoïque de formule

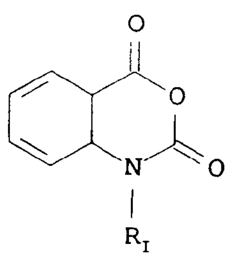

(IV)

dans lequel $R_I$ est tel que défini pour (I) pour obtenir la diphénylcétone de formule

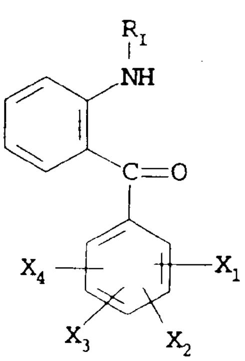

(V)

dans laquelle $X_1$, $X_2$, $X_3$, $X_4$, et $R_I$ sont tels que définis pour (I) que l'on fait réagir ensuite avec une halogéno-

cétone de formule

$$Hal\text{-}CH_2COCl$$

dans laquelle Hal représente de préférence un atome de brome ou de chlore,puis que l'on cyclise, par exemple en présence d'ammoniac, pour obtenir la benzodiazépine de formule :

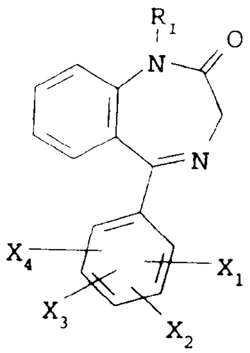

(VI)

dans laquelle $X_1$, $X_2$, $X_3$, $X_4$ et $R_I$ sont tels que définis pour (I) étant entendu que si $R_I$ est un alkyle en ($C_2$-$C_4$) le composé (VI) peut aussi être préparé par alkylation de l'amide (VI avec $R_I$ = H), par action d'un dérivé AlkI dans lequel Alk représente un alkyle en $C_2$-$C_4$, puis on prépare l'oxime du composé (VI) en faisant réagir le tert-butylate de potassium et le nitrile d'isoamyle, $(CH_3)_2$ CH-$CH_2$-$CH_2$-ONO, pour obtenir la benzodiazépine de formule

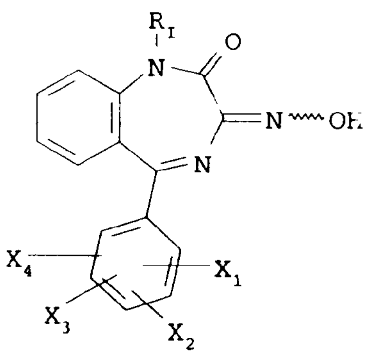

(VII)

dans laquelle $R_I$, $X_1$, $X_2$, $X_3$ et $X_4$ sont tels que définis pour (I) pour réduire ensuite la fonction oxime par l'hydrogène en présence d'un catalyseur comme le ruthénium sur charbon pour obtenir l'aminobenzodiazépin-2-one de formule

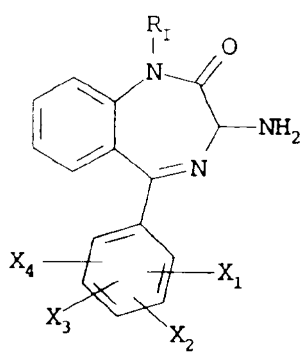

(II)

dans laquelle $X_1$, $X_2$, $X_3$, $X_4$ et $R_I$ sont tels que définis pour (I) pour la soumettre à l'action

. soit d'un acide de formule

$$R_{IIa}COOH$$

ou d'une de ses formes activées dans lequel $R_{IIa}$ est tel que défini pour (I)

. soit d'un phénylisocyanate de formule

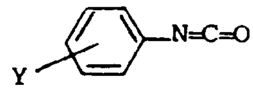

dans lequel Y est tel que défini pour (I), pour obtenir les composés (I) selon l'invention ou un de leurs sels.

Les intermédiaires de formule (II), (V), (VI) et (VII) sont des composés nouveaux faisant partie de l'invention. Les stéréoisomères et sels d'addition des composés de formule (II) font également partie de l'invention.

Les 3-aminobenzodiazépin-2-one (II) sont préparées selon des méthodes connues et décrites dans la littérature, par exemple, selon la demande de brevet WO/9307130 par réduction de l'oxime (VII) par hydrogénation catalytique ou réduction en utilisant un métal adéquat sous des conditions acides. Les catalyseurs d'hydrogénation adéquats incluent par exemple des catalyseurs de métaux nobles tels que ruthénium ou rhodium qui peuvent être fixés sur du charbon, par exemple. Cette réaction est menée de préférence dans un solvant organique adéquat tel qu'un alcool, par exemple le méthanol, à des températures de 60-70°C, préférentiellement à 60°C. Les méthodes de réduction adéquates utilisant des métaux incluent l'usage de zinc et l'acide trifluoroacétique dans un solvant adéquat tel que l'acide acétique à environ 40-50°C.

Les oximes (VII) sont préparées par réaction de l'isoamylnitrite en présence d'une base telle que des alcoolates de métaux alcalins,par exemple le tert-butylate de potassium,selon par exemple M.G. BOCK et al.,J.Org.Chem.1987,52,3232-3239.

Les benzodiazépines (VI) sont obtenues selon M.G. BOCK et al., J. Org. Chem., 1987, *52*, 3232-3239 par cyclisation entre une halogénocétone Hal-$CH_2$-COCl et une diphénylcétone de formule (V) également préparée selon BOCK et al. La diphénylcétone (V) est obtenue par condensation d'un anhydride isatoïque de formule (IV) dans lequel $R_I$ représente l'hydrogène ou un méthyle.

Lorsque $R_I$ est un alkyle en $C_2$-$C_4$, les benzodiazépines de formule (VI) sont préparées par alkylation de (VI, avec $R_I$ = H) en présence d'un hydrure alcalin tel que l'hydrure de sodium dans un solvant organique tel que le diméthylformamide en présence d'un agent alkylant AlkX dans lequel Alk représente un alkyle en $C_2$-$C_4$ et X représente un halogène de préférence l'iode, selon EP-167919.

Les 3-aminobenzodiazépin-2-one (II) peuvent également être préparées par application ou adaptation de méthodes décrites dans les brevets WO 93/07130, EP-167919, EP-540039 ou selon B.E. EVANS, J. Med. Chem., 1988, *31*, 2235.

Une autre alternative à la préparation des 3-aminobenzodiazépin-2-one, en particulier pour celles dont le phényle en position 5 est tétrasubstitué consiste en une adaptation, à ces composés, de la méthode décrite par M.G. BOCK et al., J. Org. Chem., 1987, *52*, 3232 . La préparation de la diphénylcétone peut être adaptée selon J.R. LEWIS et al., Tetrahedron, 1981, *37*, 209 par réaction de la 2-méthyl-1,3-benzoxazin-4-one avec les aryllithiens.

La préparation des composés optiquement purs de formule (I) a été réalisée en utilisant et en adaptant le procédé utilisé par M.G. BOCK et al., J. Org. Chem., 1987, 52, 3232-3239 et selon les préparations ci-après selon le SCHEMA 2 suivant :

**SCHEMA 2**

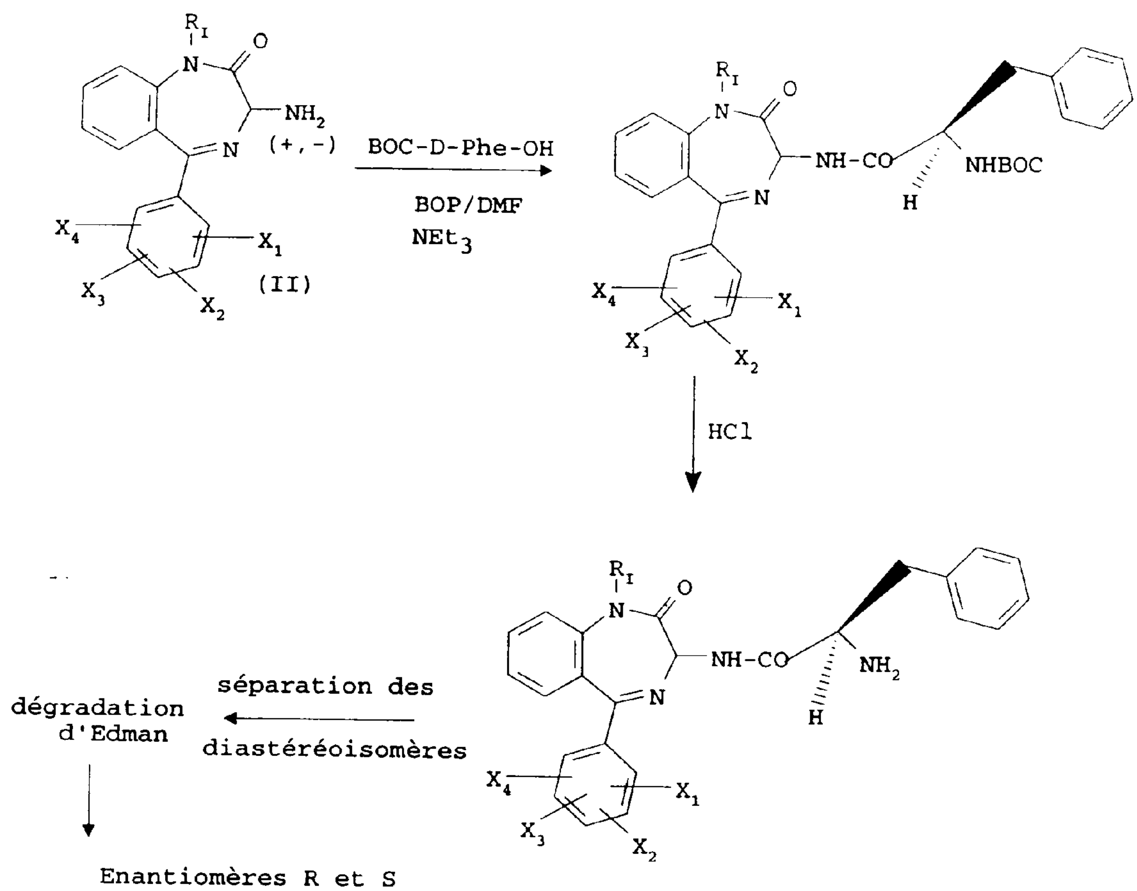


L'invention a également pour objet selon un autre de ses aspects des médicaments comprenant les composés (I) ci-dessus.

Les composés ont fait l'objet d'études de mesures d'affinité *in vitro* concernant les récepteurs CCKA.

Celle-ci est comprise entre 0,5 et 100 nM, avec une sélectivité pour les récepteurs CCKA versus les récepteurs CCKB qui peut atteindre un facteur 10 000 pour le composé de l'EXEMPLE 1, par exemple.

D'autres composés selon l'invention (composés des EXEMPLES 6 et 7) présentent une affinité pour le récepteur CCKB identique ou supérieure à celle obtenue pour le récepteur CCKA.

Une étude de l'effet agoniste vis-à-vis des récepteurs CCKA a été mise en évidence par mesure de la sécrétion d'amylase réalisée comme suit. Les acini pancréatiques sont obtenus par digestion enzymatique (collagénase) de pancréas de rat soumis à un jeûne de 18 heures. Des aliquotes (485 µl) sont incubées à 37°C pendant 30 minutes en présence de concentrations croissantes d'agoniste selon Jensen et al., J. Biol., Chem., 1982, *257*, (10), 5554. L'incubation est arrêtée par une centrifugation de 15 secondes. Le surnageant est conservé dans un bain de glace pour mesurer, le taux d'amylase selon la technique de CESKA et al., Clin., Chim., Acta, 1969, *26*, 437 (réactif phadebas). Les composés à tester sont dissous dans du diméthylsulfoxyde puis dans du tampon d'incubation.

Les composés selon l'invention se comportent comme des agonistes des récepteurs CCK avec des $CE_{50}$ (concentration de produit qui induit 50 % de la réponse maximale obtenue avec la CCK elle-même) de l'ordre de la nanomole.

L'effet agoniste démontré dans ce modèle *in vitro* a été confirmé dans un modèle *in vivo* de vidange biliaire chez la souris à jeun selon un protocole décrit par D. GULLY et al., European J. Pharmacol., 1993, *232*, 13-19. Les composés sont administrés par voie orale une heure avant le sacrifice. La vésicule biliaire est prélevée puis pesée et les résultats exprimés en mg/kg de poids corporels révèlent pour le composé de l'EXEMPLE 10, par exemple, une $DE_{50}$ de 0,3 mg/kg.

Par conséquent, les composés de formule (I) sont utilisés, en tant qu'agoniste des récepteurs de la CCK, pour la préparation de médicaments destinés au traitement de certains troubles du comportement alimentaire, de l'obésité, du diabète, dans les troubles du comportement émotionnel, sexuel et mnésique, dans les psychoses et notamment la schizophrénie, dans la maladie de Parkinson et dans divers troubles de la sphère gastrointestinale.

Les composés de formule (I) sont peu toxiques ; leur toxicité est compatible avec leur utilisation comme médicament pour le traitement des troubles et des maladies ci-dessus.

Les composés de formule (I), peuvent être formulés dans des compositions pharmaceutiques pour l'administration aux mammifères, y compris l'homme, pour le traitement des maladies susdites.

La posologie, variable selon le traitement et selon l'affection en cause peut s'échelonner, par exemple, entre 0,05 et 100 mg par jour chez l'adulte par voie orale. Le présente invention a également pour objet les compositions pharmaceutiques qui contiennent à titre de principe actif un des composés ci-dessus. Ces compositions sont réalisées de façon à pouvoir être administrées par la voie digestive ou parentérale.

Dans les compositions pharmaceutiques de la présente invention pour l'administration orale, sublinguale, sous-cutanée, intramusculaire, intraveineuse, transdermique, locale ou rectale, l'ingrédient actif peut être administré sous formes unitaires d'administration, en mélange avec des supports pharmaceutiques classiques, aux animaux et aux êtres humains. Les formes unitaires d'administration appropriées comprennent les formes par voie orale telles que les comprimés, les gélules, les poudres, les granules et les solutions ou suspensions orales, les formes d'administration sublinguale et buccale, les formes d'administration sous-cutanée, intramusculaire, intraveineuse, intranasale ou intraoculaire et les formes d'administration rectale.

Lorsque l'on prépare une composition solide sous forme de comprimés, on mélange l'ingrédient actif principal avec un véhicule pharmaceutique tel que la gélatine, l'amidon, le lactose, le stéarate de magnésium, le talc, la gomme arabique ou analogues. On peut enrober les comprimés de saccharose ou d'autres matières appropriées ou encore on peut les traiter de telle sorte qu'ils aient une activité prolongée ou retardée et qu'ils libèrent d'une façon continue une quantité prédéterminée de principe actif.

On obtient une préparation en gélules en mélangeant l'ingrédient actif avec un diluant et en versant le mélange obtenu dans des gélules molles ou dures.

Une préparation sous forme de sirop ou d'élixir peut contenir l'ingrédient actif conjointement avec un édulcorant, acalorique de préférence, du méthylparaben et du propylaraben comme antiseptique, ainsi qu'un agent donnant du goût et un colorant approprié.

Les poudres ou les granules dispersibles dans l'eau peuvent contenir l'ingrédient actif en mélange avec des agents de dispersion ou des agents mouillants, ou des agents de mise en suspension, comme la polyvinylpyrrolidone, de même qu'avec des édulcorants ou des correcteurs du goût.

Pour une administration rectale, on recourt à des suppositoires qui sont préparés avec des liants fondant à la température rectale, par exemple du beurre de cacao ou des polyéthylèneglycols.

Pour une administration parentérale, intranasale ou intraoculaire, on utilise des suspensions aqueuses, des solutions salines isotoniques ou des solutions stériles et injectables qui contiennent des agents de dispersion et/ou des agents mouillants pharmacologiquement compatibles, par exemple le propylèneglycol ou le butylène glycol.

Le principe actif peut être formulé également sous forme de microcapsules, éventuellement avec un ou plusieurs supports ou additifs.

La composition peut être sous forme de dose unitaire comprenant de 0,05 à 100 mg de principe actif.

Dans ce qui suit, on décrit des exemples de mise en oeuvre de l'invention, ainsi que les procédés de préparation de certains intermédiaires de synthèse. Les points de fusion, F, indiqués ont été déterminés en capillaire et sont exprimées en degrés Celsius.

Les exemples suivants, donnés à titre non limitatif, illustrent l'invention.

**PREPARATION I**

(2,6-Diméthoxy-4-méthylphényl)-(2-méthylaminophényl)cétone (Composé 1).

A une solution de 30 g de 3,5-diméthoxytoluène dans 400 ml de tétrahydrofurane, on ajoute à une température voisine de -20°C, 129 ml de butyllithium 1,6M dans l'hexane. L'introduction terminée, le mélange réactionnel est ramené à une température de 0°C puis agité pendant 1 heure. On refroidit à -20°C et 36,67 g d'anhydride N-méthylisatoïque sont ajoutés par portions. On laisse revenir à température ambiante et agite durant 2 heures puis on ajoute 500 ml d'eau et extrait à l'acétate d'éthyle. La phase organique est lavée à l'eau, séchée sur sulfate de sodium et évaporée sous vide. Le résidu est purifié par chromatographie sur gel de silice, éluant: toluène-acétate d'éthyle-9/1. Les fractions de produit pur sont concentrées sous vide et le résidu est cristallisé dans l'éther isopropylique, filtré et séché. On obtient des cristaux beiges qui fondent à 215°C; Rendement =

78 %.

De la même manière, on peut aussi préparer les composés suivants :

(2,4,6-Triméthoxyphényl)-(2-méthylaminophényl)cétone ; F = 190°C; Rendement = 86 % (Composé 2).

(2,3.6-Triméthoxy-4-méthylphényl)-(2-méthylaminophényl)cétone ; F = 141°C ; Rendement = 81 % (Composé 3).

**PREPARATION II**

N-[2-(2,6-diméthoxy-4-méthylbenzoyl)phényl]-N-méthylbromoacétamide (Composé 4).

A un mélange de 10 g de (2,6-diméthoxy-4-méthylphényl)-(2-méthylaminophényl)cétone dans 100 ml de dichlorométhane et de 20 ml d'eau, refroidi à -10°C, on ajoute goutte à goutte 6,34 g de chlorure de bromoacétyle dans 20 ml de dichlorométhane. On laisse revenir le mélange réactionnel à température ambiante puis on agite vivement pendant 2 heures. On décante, et successivement lave la phase organique à l'eau, sèche sur sulfate de sodium et évapore à sec pour obtenir des cristaux beiges qui fondent à 121°C ; Rendement = 99 %.

De la même façon, on peut aussi préparer les composés ci-après :

N-[2-(2,4,6-Triméthoxybenzoyl)phényl]-N-méthylbromoacétamide ; F = 118°C ; Rendement = 92 % (Composé 5).

N-[2-(2,3,6-Triméthoxy-4-méthylbenzoyl) phényl]-N-méthylbromoacétamide ; huile, Rendement = 100 % (Composé 6).

**PREPARATION III**

1,3-dihydro-5-(2,6-diméthoxy-4-méthylphényl)-1-méthyl-1,4-benzodiazépin-2-one (Composé 7).

On dissout 14 g de N-[2-(2,6-diméthoxy-4-méthylbenzoyl) phényl]-N-méthylbromoacétamide dans 300 ml de méthanol refroidi à -10°C et fait buller de l'ammoniac pendant 2 heures. On chauffe alors le mélange réactionnel à reflux pendant 3 heures puis évapore à sec. Le résidu est repris par du dichlorométhane puis successivement, on lave la phase organique à l'eau, sèche sur sulfate de sodium et évapore à sec. Le résidu est purifié par chromatographie sur colonne de gel de silice, éluant : dichlorométhane-méthanol - 95/5.

La concentration des fractions de produit pur fournit des cristaux blancs qui fondent à 200°C ; Rendement = 89 %.

On prépare de la même façon

la 1,3-dihydro-5-(2,4,6-triméthoxyphényl)-1-méthyl-1,4-benzodiazépin-2-one, F = 161°C ; Rendement = 89 %. (Composé 8), et

la 1,3-dihydro-5-(2,3,6-triméthoxy-4-méthylphényl)-1-méthyl-1,4-benzodiazépin-2-one, F = 180°C ; Rendement = 84 % (Composé 9).

**PREPARATION IV**

1,3-dihydro-5-(2,6-diméthoxy-4-méthylphényl)-3-hydroxyimino-1-méthyl-1,4-benzodiazépin-2-one (Composé 10).

A une suspension de 9 g de 1,3-dihydro-5-(2,6-diméthoxy-4-méthylphényl)-1-méthyl-1,4-benzodiazépin-2-one dans 300 ml de toluène, on ajoute par portions en refroidissant à -20°C, 7,8 g de *tert*-butylate de potassium puis on laisse remonter lentement la température à 0°C pendant 35 minutes, refroidit à -20°C et ajoute goutte à goutte 3,9 g de nitrite d'isoamyle dans 50 ml de toluène. Le mélange réactionnel est ensuite agité à 0°C pendant 90 minutes puis successivement on verse le mélange réactionnel sur de l'eau, ajuste à pH = 4 avec de l'acide acétique, extrait à l'acétate d'éthyle, lave la phase organique à l'eau, sèche sur sulfate de sodium et évapore à sec. On cristallise le résidu dans l'éther isopropylique, filtre et sèche pour obtenir des cristaux beiges qui fondent à 150°C ; Rendement = 76 %.

De la même façon, on prépare la 1,3-dihydro-5-(2,4,6-triméthoxyphényl)-1-méthyl-1,4-benzodiazépin-2-one, F = 155°C ; Rendement = 80 % (Composé 11)

la 1,3-dihydro-5-(2,3,6-triméthoxy-4-méthylphényl)-1-méthyl-1,4-benzodiazépin-2-one, F = 144°C ; Rendement = 83 % (Composé 12).

**PREPARATION V**

3-amino-1,3-dihydro-5-(2,6-diméthoxy-4-méthylphényl)-1-méthyl-1,4-benzodiazépin-2-one (Composé 13).

Dans un autoclave, on met 6,4 g de 1,3-dihydro-5-(2,6-diméthoxy-4-méthylphényl)-3-hydroxyimino-1-méthyl-1,4-benzodiazépin dans 180 ml de méthanol avec 1,32 g de ruthénium/C à 5 %, on chauffe l'autoclave à 60°C et hydrogène sous une pression d'hydrogène de 6 bars pendant 22 heures puis successivement , refroidit le mélange réactionnel, filtre le catalyseur et évapore à sec. Le résidu est repris par de l'acétone et le produit est purifié par salification par l'intermédiaire de son oxalate. On obtient des cristaux beiges d'oxalate du produit attendu qui fondent à 170°C ; Rendement = 80 %.

De la même façon, on prépare l'oxalate de 3-amino-1,3-dihydro-5-(2,4,6-triméthoxyphényl)-1-méthyl-1,4-benzodiazépin-2-one, F = 155°C ; Rendement = 83 % (Composé 14), et

la 3-amino-1,3-dihydro-5-(2,3,6-triméthoxy-4-méthylphényl)-1-méthyl-1,4-benzodiazépin-2-one, que l'on obtient sous forme d'huile ; Rendement = 75 % (Composé 15).

SEPARATION DES DIASTEREOISOMERES DES COMPOSES DE FORMULE (II)

**PREPARATION VI**

**Etape 1**

On dissout 1,73 g de 3-amino-1,3-dihydro-5-(2,6-diméthoxy-4-méthylphényl)-1-méthyl-1,4-benzodiazépin-2-one dans 30 ml de diméthylformamide en présence de 1,42 g de BOC-D-phénylalanine puis on refroidit à 5°C et ajoute 1,07 ml de triéthylamine puis 2,4 g de BOP. On laisse ensuite revenir le mélange réactionnel à température ambiante et l'abandonne sous agitation pendant une nuit. On le verse ensuite sur de l'eau et extrait à l'acétate d'éthyle. La phase organique est lavée à l'eau, décantée, séchée sur $Na_2SO_4$, filtrée et concentrée sous vide. La 2-*tert*-butoxycarbonylamino-N-[2,3-dihydro-5-(2,6-diméthoxy-4-méthylphényl)-1-méthyl-2-oxo-1*H*-1,4-benzodiazépin-3-yl]-3-phénylpropionamide, résidu huileux obtenu avec un rendement quantitatif, est utilisé tel quel pour l'étape suivante (Composé 16).

**Etape 2**

On dissout 4,6 g de 2-*tert*-butoxycarbonylamino-N-[2,3-dihydro-5-(2,6-diméthoxy-4-méthylphényl)-1-méthyl-2-oxo-1*H*-1,4-benzodiazépin-3-yl]-3-phénylpropionamide obtenu précédemment dans 30 ml d'acétate d'éthyle, refroidit le mélange à 0°C puis ajoute goutte à goutte 30 ml d'une solution 5N de chlorure d'hydrogène dans l'acétate d'éthyle.

L'introduction terminée on laisse revenir à température ambiante et agite la solution pendant 3 heures. Le mélange réactionnel est versé dans de l'éther éthylique, on sépare le précipité formé par filtration, puis successivement on le lave à l'éther éthylique et sèche pour obtenir la 2-amino-N-[2,3-dihydro-5-(2,6-diméthoxy-4-méthylphényl)-1-méthyl-2-oxo-1*H*-1,4-benzodiazépin-3-yl]-3-phénylpropionamide sous forme de cristaux blancs avec un rendement de 90 % ; F = 165°C (Composé 17).

**Etape 3**

On sépare les diastéréoisomères obtenus à l'étape précédente par H.P.L.C. préparative. La séparation des diastéréoisomères se fait sur un appareil PROCHROM LC50 à compression dynamique axiale. La phase stationnaire utilisée est une phase reverse KROMASIL de porosité 100Å. L'élution a lieu en mode isocratique avec 65 % de (eau + acide trifluoroacétique à 1 %) et 35 % de (acétonitrile - eau - 90/10 avec 0,8 ‰ d'acide trifluoroacétique) à un débit de 120 ml/minute et avec une détection UV à 220 nm. Avec 3,7 g de racémique provenant de l'étape précédente, on obtient, après lyophilisation et basification chacun des deux stéréoisomères sous forme de mousse avec un rendement de séparation de 45 %.

**PREPARATION VII**

Synthèse de l'énantiomère dextrogyre et lévogyre du 3-amino-1,3-dihydro-5-(2,6-diméthoxy-4-méthylphényl)-1-méthyl-1,4-benzodiazépin-2-one.

Les deux énantiomères sont obtenus en deux étapes à partir des deux diastéréoisomères isolés précédemment en utilisant la méthode de dégradation classique d'Edman selon P. EDMAN, Acta Chem. Scand., 1950, *4*, 283.

**Synthèse de l'énantiomère dextrogyre**

**Etape 1** (Composé 18).

On dissout 720 mg d'un diastéréoisomère obtenu à l'Etape 3 de la PREPARATION VI précédente dans 10 ml de dichlorométhane et ajoute 220 mg de phénylisothiocyanate. On chauffe le mélange à reflux pendant 1 heure puis évapore le mélange réactionnel sous vide et cristallise le résidu dans l'éther isopropylique. On obtient des cristaux qui fondent à 140°C ; Rendement = 95 %.

**Etape 2**

On dissout 860 mg du dérivé thiourée obtenu à l'étape précédente dans 20 ml d'acétate d'éthyle, refroidit à une température voisine de 5°C et ajoute goutte à goutte 10 ml d'acétate d'éthyle chlorhydrique voisin de 5N. On laisse revenir à température ambiante et agite 2 heures puis évapore le mélange réactionnel et chromatographie sur colonne de gel de silice (éluant : dichlorométhane-méthanol-eau-acide acétique - 90/10/1/1). On évapore les fractions de produit pur sous vide, puis successivement on évapore, basifie le résidu, extrait au dichlorométhane, lave à l'eau et sèche. Après évaporation, on obtient des cristaux qui fondent à 139°C ; Rendement = 89 %. $[\alpha]_D^{20}$= + 140 (c = 0,2 dans le $CH_3OH$).

**Synthèse de l'énantiomère lévogyre** (Composé 19)

L'énantiomère lévogyre est obtenu de façon identique en utilisant la dégradation d'Edman sur l'autre diastéréoisomère. On obtient des cristaux qui fondent à 139°C ; Rendement = 85 % ; $[\alpha]_D^{20}$ = - 146 (c = 0,2 dans le $CH_3OH$).

EXEMPLE 1

(I) : $R_I = CH_3$ ;

$R_{II}$ = 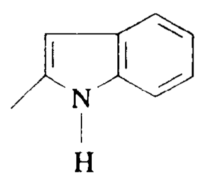 ;

$X_1$ = 2-$OCH_3$ ; $X_2$ = 6-$OCH_3$ ; $X_3$ = 4-$CH_3$ ; $X_4$ = H

On dissout 0,47 ml de pyridine dans 10 ml de dichlorométhane, refroidit à -5°C et ajoute goutte à goutte 0,68 ml de chlorure de thionyle, laisse le mélange 20 minutes à la même température puis ajoute par portions 208 mg d'acide indole-2-carboxylique et agite 30 minutes. On ajoute par portions 400 mg de 3-amino-1,3-dihydro-5-(2,6-diméthoxy-4-méthylphényl)-1-méthyl-1,4-benzodiazépin-2-one puis laisse revenir à température ambiante et agite le mélange réactionnel pendant 16 heures. On ajoute de l'eau au mélange réactionnel, filtre le précipité formé et séche pour obtenir des cristaux blancs de N-[2,3-dihydro-5-(2,6-diméthoxy-4-méthylphényl)-1-méthyl-2-oxo-1*H*-1,4-benzodiazépin-3-yl]-1*H*-2-indolecarboxamide qui fondent avec décomposition à 310°C ; Rendement = 79 %.

EXEMPLE 2

(I) : $R_I$=$CH_3$ ;

$R_{II}$ = 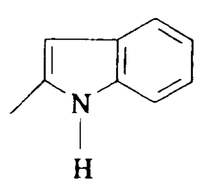 ;

$X_1$ = 2-$OCH_3$ ; $X_2$ = 6-$OCH_3$ ; $X_3$ = 4-$OCH_3$ ; $X_4$ = H

En procédant selon l'EXEMPLE 1 et en utilisant la 3-amino-1,3-dihydro-5-(2,4,6-triméthoxyphényl)-1-méthyl-1,4-benzodiazépin-2-one on obtient la N-[2,3-dihydro-5-(2,4,6-triméthoxyphényl)-1-méthyl-2-oxo-1*H*-1,4-benzodiazépin-3-yl]-1*H*-2-indole-carboxamide ; F = 297°C ; Rendement = 87%.

EXEMPLE 3

(I) : $R_I$ = $CH_3$ ;

$R_{II}$ = 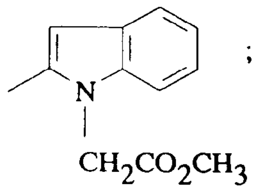 ;

$X_1$ = 2-$OCH_3$ ; $X_2$ = 6-$OCH_3$ ; $X_3$ = 4-$CH_3$ ; $X_4$ = H

A une solution de 0,37 g de 3-amino-1,3-dihydro-5-(2,6-diméthoxy-4-méthylphényl)-1-méthyl-1,4-benzodiazépin-2-one dans 10 ml de diméthylformamide refroidi à 5°C, on ajoute 0,28 g d'acide 1-méthoxycarbonylméthyl-1*H*-indole-2-carboxylique, 578 mg de R.O.P. et 0,303 ml de triéthylamine. Le mélange réactionnel est agité une nuit à température ambiante puis versé sur de l'eau et extrait à l'acétate d'éthyle. On lave successivement la phase organique à l'eau, sèche sur sulfate de sodium et évapore à sec. Les cristaux obtenus sont recristallisés dans un mélange éther isopropylique isopropanol. On obtient des cristaux blancs de {N-[2,3-dihydro-5-(2,6-diméthoxy-4-méthylphényl)-1-méthyl-2-oxo-1*H*-1,4-benzodiazépin-3-yl]-2-aminocarbonylindol-1-yl}acétate de méthyle qui fondent à 242°C ; Rendement = 77 %.

EXEMPLE 4

(I) : $R_I$ = $CH_3$ ;

$R_{II}$ = 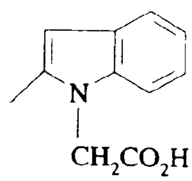 ;

$X_1$ = 2-$OCH_3$ ; $X_2$ = 6-$OCH_3$ ; $X_3$ = 4-$CH_3$ ; $X_4$ = H

On dissout 220 mg de N-[2,3-dihydro-5-(2,6-diméthoxy-4-méthylphényl)-1-méthyl-2-oxo-1*H*-1,4-benzodiazépin-3-yl-2-aminocarbonyl-1*H*-indol-1-yl]acétate de méthyle dans 10 ml de méthanol et ajoute goutte à goutte 1 ml d'hydroxyde de sodium 1N puis agite une nuit à température ambiante. On reprend le mélange par de l'eau et extrait à l'éther éthylique. On acidifie la phase aqueuse jusqu'à pH = 3 avec de l'hydrogénosulfate de potassium et extrait au dichlorométhane, sèche sur sulfate de sodium et évapore à sec. On cristallise l'acide, [N-[2,3-dihydro-5-(2,6-diméthoxy-4-méthylphényl)-1-méthyl-2-oxo-1*H*-1,4-benzodiazépin-3-yl]-2-aminocarbonyl-1*H*-indol-1-yl] acétique obtenu, dans l'éther isopropylique pour obtenir des cristaux blancs qui fondent à 138°C ; Rendement = 75 %.

EXEMPLE 5

(I) : $R_I$ = $CH_3$ ;

$R_{II}$ = 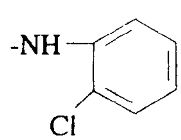 ;

$X_1$ = 2-$OCH_3$ ; $X_2$ = 6-$OCH_3$ ; $X_3$ = 4-$CH_3$ ; $X_4$ = H

A 56 mg de 3-amino-1,3-dihydro-5-(2,6-diméthoxy-4-méthylphényl)-1-méthyl-1,4-benzodiazépin-2-one dans 10 ml de dichlorométhane, on ajoute 0,022 ml de 2-chlorophénylisocyanate puis agite le mélange réactionnel à température ambiante pendant une nuit. On concentre sous vide et le résidu est cristallisé dans l'éther isopropylique pour fournir la 3-(2-chlorophényluréido)-1,3-dihydro-5-(2,6-diméthoxy-4-méthylphényl)-1-méthyl-1,4-benzodiazépin-2-one ; F = 260°C ; Rendement = 91 %.

EXEMPLE 6

(I) : $R_I = CH_3$ ;

$R_{II}$ = 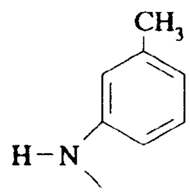 ;

$X_1$ = 2-$OCH_3$ ; $X_2$ = 6-$OCH_3$ ; $X_3$ = 4-$CH_3$ ; $X_4$ = H

En utilisant la 3-amino-1,3-dihydro-5-(2,6-diméthoxy-4-méthylphényl)-1-méthyl-1,4-benzodiazépin-2-one comme précédemment et en la condensant comme précédemment avec le 3-méthylphénylisocyanate on obtient la 1,3-dihydro-5-(2,6-diméthoxy-4-méthylphényl)-1-méthyl-3-(3-méthylphényluréido)-1,4-benzodiazépin-2-one ; F = 260°C ; Rendement = 94 %.

EXEMPLE 7

(I) : $R_I = CH_3$ ;

$R_{II}$ = 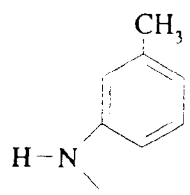 ;

$X_1$ = 2-$OCH_3$ ; $X_2$ = 4-$OCH_3$ ; $X_3$ = 6-$OCH_3$ ; $X_4$ = H

En condensant la 3-amino-1,3-dihydro-1-méthyl-5-(2,4,6-triméthoxyphényl)-1,4-benzodiazépin-2-one avec le 3-méthylphénylisocyanate on obtient la 1,3-dihydro-1-méthyl-3-(3-méthylphenyluréido)-5-(2,4,6-triméthoxyphényl)-1,4-benzodiazépin-2-one ; F = 240°C, Rendement = 94 %.

EXEMPLE 8

(I) : $R_I = CH_3$ ;

$R_{II}$ = 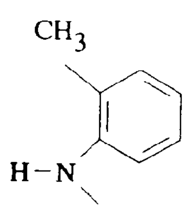 ;

$X_1$ = 2-$OCH_3$ ; $X_2$ = 4-$OCH_3$ ; $X_3$ = 6-$OCH_3$ ; $X_4$ = H

En procédant selon l'EXEMPLE 7 et en utilisant le 2-méthylphénylisocyanate on obtient la 1,3-dihydro-1-méthyl-3-(2-méthylphényluréido)-5-(2,4,6-triméthoxyphényl)-1,4-benzodiazépin-2-one ; F = 250°C ; Rendement = 92 %.

EXEMPLE 9

(I) : $R_I = CH_3$ ;

$R_{II}$ = 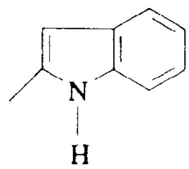 ;

$X_1$ = 2-$OCH_3$ ; $X_2$ = 4-$CH_3$ ; $X_3$ = 6-$OCH_3$ ; $X_4$ = H énantiomère lévogyre.

A une solution de 0,38 g de l'énantiomère lévogyre de 3-amino-1,3-dihydro-5-(2,6-diméthoxy-4-méthylphényl)-1-méthyl-1,4-benzodiazépin-2-one dans 10 ml de diméthylformamide, refroidi à 5°C, on ajoute 0,52 g de B.O.P. et 0,19 g d'acide indole-2-carboxylique puis 0,23 ml de triéthylamine. Le mélange réactionnel est agité une nuit à température ambiante puis versé sur de l'eau et extrait à l'acétate d'éthyle, séché sur sulfate de sodium et évaporé à sec. Le produit est purifié par chromatographie sur colonne de gel de silice (éluant: dichlorométhane-méthanol : 95/5). Les fractions de produit pur sont évaporées à sec et le résidu est cristallisé dans l'éther isopropylique. On obtient des cristaux blancs de (-)N-[2,3-dihydro-5-(2,6-diméthoxy-4-méthylphényl)-1-méthyl-2-oxo-1*H*-1,4-benzodiazépin-3-yl]-1*H*-indol-2-carboxamide qui fondent à 135°C ; Rendement = 81 % ; $[\alpha]_D^{20}$ = -408 (c = 0,35 dans HClIN-$CH_3OH$-50/50).

EXEMPLE 10

(I) : $R_I = CH_3$ ;

$R_{II}$ = 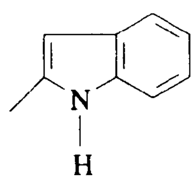 ;

$X_1$ = 2-$OCH_3$ ; $X_2$ = 4-$CH_3$ ; $X_3$ = 6-$OCH_3$ ; $X_4$ = H énantiomère dextrogyre.

Ce composé est obtenu de façon identique à l'autre énantiomère (EXEMPLE 9) à partir de l'énantiomère dextrogyre de 3-amino-1,3-dihydro-5-(2,6-diméthoxy-4-méthylphényl)-1-méthyl-1,4-benzodiazépin-2-one. On obtient des cristaux blancs de (+)-N-[2,3-dihydro-5-(2,6-diméthoxy-4-méthylphényl)-1-méthyl-2-oxo-1*H*-1,4-benzodiazépin-3-yl]-1*H*-indol-2-carboxamide dextrogyre qui fondent à 135°C ; Rendement = 78 % ; $[\alpha]_D^{20}$ = + 412 (c = 0,32 dans le HClIN-$CH_3OH$-50/50)

EXEMPLE 11

(I) : $R_I = CH_3$ ;

$R_{II}$ = 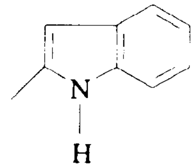 ;

$X_1$ = 2-$OCH_3$ ; $X_2$ = 3-$OCH_3$ ; $X_3$ = 6-$OCH_3$ ; $X_4$ = 4-$CH_3$

En procédant selon l'EXEMPLE 3, à partir de la 3-amino-1,3-dihydro-5-(2,3,6-triméthoxy-4-méthylphényl)-1-méthyl-1,4-benzodiazépin-2-one et d'acide indole-2-carboxylique, on obtient des cristaux de N-[2,3-dihydro-5-(2,3,6-triméthoxy-4-méthylphényl)-1-méthyl-2-oxo-1*H*-1,4-benzodiazépin-3-yl]-1*H*-2-indolecarboxa mide fondant à 290°C ; Rendement = 68 %.

**Revendications**

1. Composé de formule (I) :

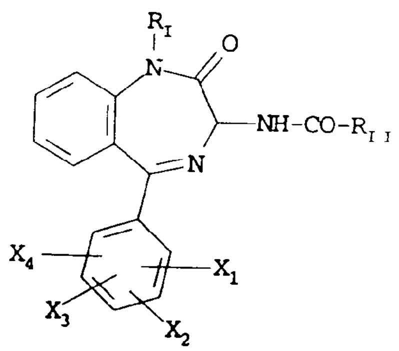

(I)

dans laquelle

- $R_I$ représente (i) l'hydrogène ; (ii) un $(C_1\text{-}C_4)$ alkyle; (iii) un groupe $-CH_2\text{-}CHOH\text{-}(CH_2)_m\text{-}Z$ dans lequel m est 0 ou 1 et Z représente un groupe alkyle en $(C_1\text{-}C_4)$; cycloalkyle en $(C_3\text{-}C_8)$; aryle éventuellement substitué par $(C_1\text{-}C_4)$ alkyle, $(C_1\text{-}C_4)$ alkoxy ou halogène;ou Z représente un hétérocycle saturé ou non, éventuellement substitué par $(C_1\text{-}C_4)$ alkyle, $(C_1\text{-}C_4)$ alkoxy ou halogène; ou $R_I$ représente (iv) un groupe $-(CH_2)_nCOR_o$ dans lequel
  n représente 1 à 3,
  $R_o$ représente $OR_2$ ou $NR_2R_3$, $R_2$ et $R_3$ identiques ou non représentant H ou $(C_1\text{-}C_4)$ alkyle ou bien $R_2$ et $R_3$ constituant ensemble avec l'atome d'azote auquel ils sont liés un hétérocycle saturé ou non;
- $R_{II}$ représente soit (i) un hétérocycle aromatique azoté, $R_{IIa}$, choisi parmi quinoléine, isoquinoléine, benzimidazole et indole, ce dernier étant éventuellement substitué sur l'azote par un groupe W où W représente le groupe $CO\text{-}(C_1\text{-}C_4)$alkyle; ou le groupe $(CH_2)_nCOR_o$ dans lequel n et $R_o$ sont tels que définis pour $R_I$; soit (ii) un groupe

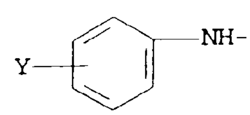

$(R_{IIb})$

dans lequel Y représente un atome d'halogène, un groupe $(C_1\text{-}C_3)$ alkyle, ou $(C_1\text{-}C_3)$ alcoxy;
- $X_1$, $X_2$ et $X_3$ sont identiques ou différents et représentent un $(C_1\text{-}C_3)$ alkyle, un $(C_1\text{-}C_3)$ alcoxy, un atome d'halogène ou un groupe trifluorométhyle;
- $X_4$ représente l'hydrogène ou est identique à $X_1$, $X_2$ ou $X_3$, leurs stéréosiomères et leurs sels d'addition.

2. Composé de formule (1), selon la revendication 1 dans lequel $R_{II}$ représente un groupe $R_{IIa}$ qui représente un indole éventuellement substitué et dans lequel W, $R_I$, $X_1$, $X_2$, $X_3$ et $X_4$ sont tels que définis pour (I) ainsi que ses sels.

3. Composé de formule (I), selon la revendication 1 dans Lequel $R_{II}$ représente un groupe $R_{IIb}$ et dans lequel Y, $R_1$, $X_1$, $X_2$, $X_3$ et $X_4$ sont tels que définis pour (I) ainsi que ses sels.

4. Composé de formule (I) selon la revendication 1, dans lequel $X_1$ est en position 2, $X_2$ est en position 4, $X_3$ est en position 6 et $X_4$ est indifféremment en position 3 ou 5 du radical phényle.

5. Composé de formule (I), selon la revendication 1 dans lequel $X_1$ représente un groupe $2\text{-}(C_1\text{-}C_3)$alcoxy, $X_2$ représente un groupe $6\text{-}(C_1\text{-}C_3)$ alcoxy et $X_3$, $X_4$, $R_I$ et $R_{II}$ sont tels que définis pour (I) ainsi que ses sels.

6. Composé de formule I selon la revendication 1, dans lequel trois au moins des substituants $X_1$, $X_2$, $X_3$ et $X_4$ représentent $(C_1\text{-}C_2)$alkyle ou $(C_1\text{-}C_2)$ alcoxy.

7. Composé de formule (I), selon l'une quelconque des revendications 1 à 4 sous forme optiquement pure.

8. Procédé de préparation des composés de formule (I), selon la revendication 1, caractérisé en ce que l'on fait réagir un dérivé benzénique substitué de formule

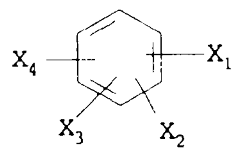

(III)

dans laquelle $X_1$, $X_2$, $X_3$ et $X_4$ sont tels que définis pour (I) avec un anhydride isatoïque de formule

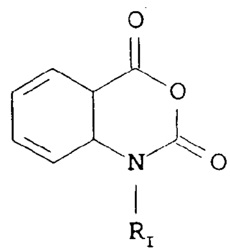

(IV)

dans lequel $R_I$ est tel que défini pour (I) pour obtenir la diphénylcétone de formule

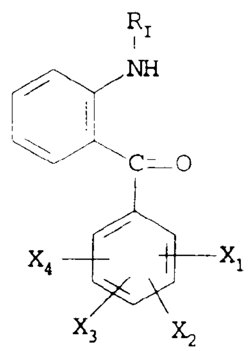

(V)

dans laquelle $X_1$, $X_2$, $X_3$, $X_4$, et $R_I$ sont tels que définis pour (I) que l'on fait réagir avec une halogénocétone de formule

$$Hal\text{-}CH_2COCl$$

dans laquelle Hal représente de préférence un atome de brome ou de chlore que l'on cyclise ensuite pour obtenir la benzodiazépine de formule

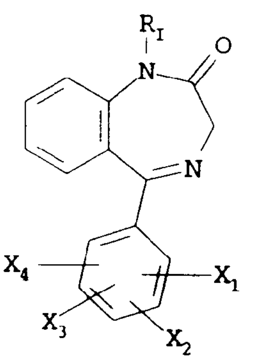

(VI)

dans laquelle $X_1$, $X_2$, $X_3$, $X_4$ et $R_I$ sont tels que définis pour (I) étant entendu que si $R_I$ est un alkyle en ($C_2$-

$C_4$), le composé (VI) peut aussi être préparé par alkylation de l'amide (VI avec $R_I$ = H), par action d'un dérivé AlkI dans lequel Alk représente un alkyle en $C_2$-$C_4$, puis on prépare l'oxime du composé (VI) en faisant réagir le *tert*-butylate de potassium et le nitrile d'isoamyle, $(CH_3)_2$ $CH$-$CH_2$-$CH_2$-ONO, pour obtenir la benzodiazépine de formule

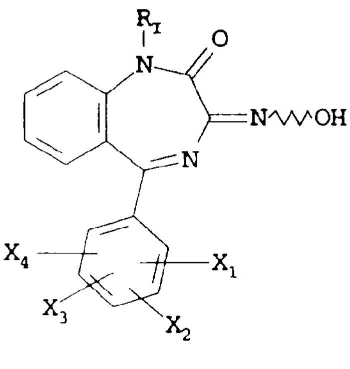

(VII)

dans laquelle $R_I$, $X_1$, $X_2$, $X_3$ et $X_4$ sont tels que définis pour (I) pour réduire ensuite la fonction oxime par l'hydrogène en présence d'un catalyseur comme le ruthénium sur charbon pour obtenir l'aminobenzodiazépin-2-one de formule

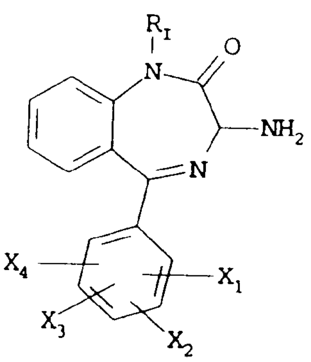

(II)

dans laquelle $X_1$, $X_2$, $X_3$, $X_4$ et $R_I$ sont tels que définis pour (I) pour la soumettre à l'action

. soit d'un acide de formule

$$R_{IIa}COOH$$

ou d'une de ses formes activées dans lequel $R_{IIa}$ est tel que défini pour (I)

. soit d'un phénylisocyanate de formule

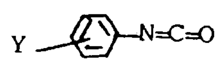

dans lequel Y est tel que défini pour (I), pour obtenir les composés (I) selon l'invention ou un de leurs sels.

**9.** Composition pharmaceutique contenant en tant que principe actif un composé selon l'une quelconque des revendications 1 à S ou un de ses sels pharmaceutiquement acceptable.

**10.** Composition pharmaceutique selon la revendication 7 sous forme d'unité de dosage.

**11.** Composition pharmaceutique selon la revendication 8, caractérisée en ce qu'elle contient de 0,05 à 100 mg de principe actif avec au moins un excipient pharmaceutique.

**12.** Composé de fommule (II)

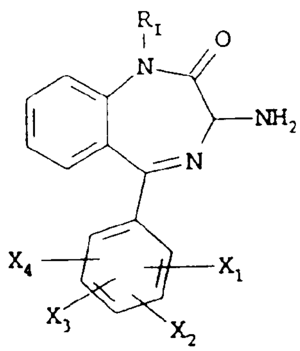

(II)

dans laquelle

- $R_I$ représente (i) l'hydrogène ; (ii) un ($C_1$-$C_4$) alkyle; (iii) un groupe $-CH_2-CHOH-(CH_2)_m-Z$ dans lequel m est 0 ou 1 et Z représente un groupe alkyle en ($C_1$-$C_4$); cycloalkyle en ($C_3$-$C_8$); aryle éventuellement substitué par ($C_1$-$C_4$) alkyle, ($C_1$-$C_4$) alkoxy ou halogène;ou Z représente un hétérocycle saturé ou non, éventuellement substitué par ($C_1$-$C_4$) alkyle, ($C_1$-$C_4$) alkoxy ou halogène; ou $R_I$ représente (iv) un groupe $-(CH_2)_mCOR_o$ dans lequel

n représente 1 à 3,

$R_o$ représente $OR_2$ ou $NR_2R_3$, $R_2$ et $R_3$ identiques ou non représentant H ou ($C_1$-$C_4$) alkyle ou bien $R_2$ et $R_3$ constituant ensemble avec l'atome d'azote auquel ils sont liés un hétérocycle saturé ou non;

$X_1$, $X_2$ et $X_3$ sont identiques ou différents et représente un ($C_1$-$C_3$) alkyle, un ($C_1$-$C_3$) alcoxy, un atome d'halogène ou un groupe trifluorométhyle; et

$X_4$ représente l'hydrogène ou est identique à $X_1$, $X_2$ ou $X_3$, leurs stéréoisomères et sels d'addition.

Office européen des brevets

# RAPPORT DE RECHERCHE EUROPEENNE

Numero de la demande
EP 95 40 0293

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.6) |
|---|---|---|---|
| D,A | EP-A-0 167 919 (MERCK & CO. INC.)<br>* le document en entier *<br>--- | 1-12 | C07D403/12<br>C07D243/24<br>C07D401/12<br>A61K31/55 |
| D,A | EP-A-0 376 849 (ROUSSEL-UCLAF)<br>* le document en entier *<br>--- | 1-12 | |
| A | CHEMICAL ABSTRACTS, vol. 72, no. 15, 13 Avril 1970, Columbus, Ohio, US;<br>abstract no. 79116k,<br>page 414 ;<br>* abrégé *<br>& JP-A-69 026 871 (TANABE SEIYAKU CO., LTD.)<br>----- | 1-12 | |
| | | | **DOMAINES TECHNIQUES RECHERCHES (Int.Cl.6)** |
| | | | C07D |

Le présent rapport a été établi pour toutes les revendications

| Lieu de la recherche | Date d'achèvement de la recherche | Examinateur |
|---|---|---|
| LA HAYE | 23 Mai 1995 | Allard, M |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)